(19) Europäisches Patentamt
European Patent Office
Office européen des brevets

(11) Veröffentlichungsnummer: **0 007 996**
**B1**

(12) # EUROPÄISCHE PATENTSCHRIFT

(45) Veröffentlichungstag der Patentschrift: **26.01.83**

(51) Int. Cl.³: **C 07 H 15/22,**
A 61 K 31/70 //C07D317/72

(21) Anmeldenummer: **79102220.5**

(22) Anmeldetag: **02.07.79**

(54) **4,6-Di-0-(Aminoglycosyl)-1,3-Diaminocyclitol-Derivate, Verfahren zu deren Herstellung und diese Verbindungen enthaltende Arzneimittel.**

(30) Priorität: **22.07.78 DE 2832268**

(43) Veröffentlichungstag der Anmeldung:
**20.02.80 Patentblatt 80/4**

(45) Bekanntmachung des Hinweises auf die Patenterteilung:
**26.01.83 Patentblatt 83/4**

(84) Benannte Vertragsstaaten:
**AT BE CH DE FR GB IT NL SE**

(56) Entgegenhaltungen:
**FR - A - 2 355 029**
**FR - A - 2 383 963**

**CHEMICAL ABSTRACTS, vol. 87, Nr. 7, 15. August 1977, Seite 503, Zusammenfassung 53534e. Columbus, Ohio, USA**

**Die Akte enthält technische Angaben, die nach dem Eingang der Anmeldung eingereicht wurden und die nicht in dieser Patentschrift enthalten sind.**

(73) Patentinhaber: **BAYER AG**
**Zentralbereich Patente, Marken und Lizenzen**
**D-5090 Leverkusen 1, Bayerwerk (DE)**

(72) Erfinder: **Stadler, Peter, Dr.**
**Ohligser Strasse 85**
**D-5657 Haan (DE)**
Erfinder: **Metzger, Karl Georg, Dr.**
**Pahlkestrasse 15**
**D-5600 Wuppertal 1 (DE)**
Erfinder: **Voss, Eckart, Dr.**
**Morgengraben 10**
**D-5000 Köln 80 (DE)**
Erfinder: **Petersen, Uwe, Dr.**
**Auf dem Forst 4**
**D-5090 Leverkusen 1 (DE)**
Erfinder: **Zeiler, Hans-Joachim, Dr.**
**Windratherstrasse 188**
**D-5620 Velbert 15 (DE)**
Erfinder: **Kabbe, Hans-Joachim, Dr.**
**Walter-Flex-Strasse 16**
**D-5090 Leverkusen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 007 996 B1

# 4,6-Di-O-(Aminoglycosyl)-1,3-Diaminocyclitol-Derivate, Verfahren zu deren Herstellung und diese Verbindungen enthaltende Arzneimittel

Die vorliegende Erfindung betrifft neue, antibakteriell wirksame Aminoglycosid-Derivate vom Typ des Sisomicins, Verfahren zu ihrer Herstellung sowie diese Verbindungen enthaltende Arzneimittel.

Aminoglykosidantibiotika sind wichtige Substanzen zur wirkungsvollen Bekämpfung bakterieller Infektionen. Das Auftreten resistenter Keime mindert jedoch in vielen Fällen ihre breite Anwendbarkeit; desweiteren können Nebenwirkungen wie Oto- und Nephrotoxizatät auftreten. Durch Derivatisierung gelingt es in einigen Fällen, diese Nachteile zu beseitigen.

Es wurde nun gefunden, daß die genannten Nachteile in besonders hohem Maße durch die erfindungsgemäßen Verbindungen der Formel I

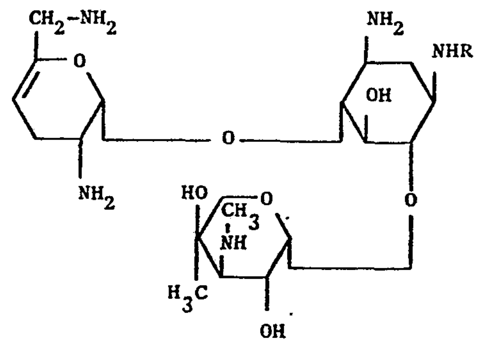

(I)

überwunden werden, wobei

R einen 2,3,4-Trihydroxypentyl-,3,4,5-Trihydroxypentyl- oder 2,3,4,5-Tetrahydroxypentylrest bedeutet.

Erfindungsgemäß bevorzugt ist das 1-N-(2,3,4,5-Tetrahydroxypentyl)-sisomicin.

Die erfindungsgemäßen Verbindungen sowie deren pharmazeutisch verwendbare Salze zeigen starke antibakterielle Eigenschaften gegen eine Vielzahl von Keimen und eine außergewöhnlich gute Verträglichkeit.

Die pharmazeutisch verwendbaren Salze leiten sich insbesondere von anorganischen oder organischen Säuren wie Schwefelsäure, Phosphorsäure, Salpetersäure, Salzsäure, Bromwasserstoffsäure, Essigsäure, Propionsäure, Ascorbinsäure, Zitronensäure usw. ab.

Als Beispiele für die erfindungsgemäßen Wirkstoffe seien genannt:

1-N-[(S,S,R)-, 1-N-[(R,R,S)-, 1-N-[(R,S,R)-, 1-N-[(S,R,S)-, 1-N-[(S,R,R)-, 1-N-[(R,S,S)-, 1-N-[(R,R,R)-, 1-N-[(S,S,S)-2,3,4,5-Tetrahydroxypentyl]-sisomicin, 1-N-[(S,R)-, 1-N-[(R,S)-, 1-N-[(R,R)- und 1-N-[(S,S)-3,4,5-Trihydroxypentyl]-sisomicin.

Erfindungsgemäß bevorzugt ist das 1-N-[(R,R,S)-2,3,4,5-Tetrahydroxypentyl]-sisomicin.

Die erfindungsgemäßen Verbindungen werden erhalten, indem man eine Verbindung der Formel II

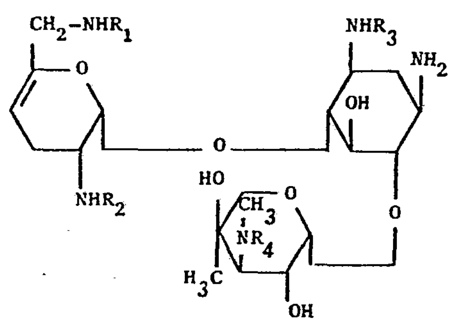

(II)

worin

$R_1$, $R_2$, $R_3$ and $R_4$ eine Gruppe —SR′ oder —CO—A′ bezeichnen, wobei

R′ für eine gegebenenfalls substituierte Phenyl-, Di- oder Triphenylmethylgruppe und

A′ für einen Rest

$-(CH_2)_{n_1}-B$ oder 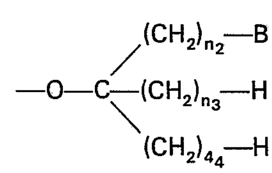

stehen,

B Wasserstoff oder eine gegebenenfalls substituierte Phenylgruppe bedeutet, und $n_1$, $n_2$, $n_3$ und $n_4$ unabhängig voneinander Zahlen von 0 bis 5 darstellen, mit einem Aldehyd der Formel III

$$HOC—Y, \qquad (III)$$

wobei
Y für einen 1,2,3-Trihydroxybutyl-, 2,3,4-Trihydroxybutyl- oder 1,2,3,4-Tetrahydroxybutylrest steht, in Gegenwart eines Wasserstoffdonor-Reduktionsmittels umsetzt und anschließend vorhandenen Schutzgruppen abspaltet.

Gegebenenfalls substituiertes Phenyl R′ ist insbesondere Phenyl oder durch ein bis drei Substituenten aus der Reihe Nitro, $C_1$—$C_4$-Alkyl, $C_1$—$C_4$-Alkoxy, $C_1$—$C_4$-alkoxycarbonyl oder Phenyl oder 1 bis 5 Halogenatome substituiertes Phenyl.

Gegebenenfalls substituiertes Phenyl B ist insbesondere Phenyl oder durch ein oder zwei Substituenten aus der Reihe Nitro, $C_1$—$C_4$-Alkyl, $C_1$—$C_4$-Alkoxy, Phenyl oder Halogen substituiertes Phenyl.

Die Verbindungen der Formel (II) werden erhalten, indem man Sisomicin beispielsweise mit o-Nitrophenylsulfensäure-p-nitrophenylester in einem inerten Lösungsmittel, gegebenenfalls unter Zusatz von Wasser, bei Temperaturen zwischen —30 und +50°C in Gegenwart einer Base umsetzt. Dabei setzt man auf ein Mol Sisomicin je nach dem, wie viel Aminogruppen geschützt werden sollen, ein bis vier Mol des Sulfensäureesters ein. Weitere Reagenzien, mit denen Schutzgruppen eingeführt werden können, sind Tritylsulfenylchlorid, o-Nitrophenylsulfenylchlorid, 2,4-Dinitrophenylsulfenylchlorid, 2,4,5-Trichlorphenylsulfenylchlorid, Pentachlorphenylsulfenylchlorid, 2,4-Dinitrophenylsulfensäure-p-nitrophenylester, 2,4,5-Trichlorphenylsulfensäure-p-nitrophenylester, Pentachlorphenylsulfensäure-p-nitrophenylester, Acetanhydrid, Acetylchlorid, Di-t-butylpyrocarbonat und Diethylpyrocarbonat.

Die Abspaltung der Sulfenylschutzgruppen kann mit schwachen Säuren erfolgen, die Abspaltung der übrigen Schutzgruppen kann mit wäßrigem Alkali- oder Erdalkalihydroxid oder mit Säuren wie Trifluoressigsäure, Perchlorsäure oder Bortrifluoridätherat erfolgen.

Die reduktive Alkylierung mit einem Aldehyd der Formel (III) in Gegenwart eines Wasserstoffdonor-Reduktionsmittels wird gewöhnlich bei Raumtemperatur in Gegenwart von Luft durchgeführt, obwohl es günstiger sein kann, die Reaktion unter Inertgas (Argon, Stickstoff) durchzuführen. Die Reaktion ist gewöhnlich sehr rasch, oft in weniger als 60 Minuten, vollendet, was durch dünnschichtchromatographische Bestimmungen festgestellt werden kann.

Wasserstoff-Donor-Reduktionsmittel, die bei diesem Verfahren Verwendung finden, umfassen Dialkylaminborane, z. B. Dimethylaminboran, Diethylaminboran und Morpholinboran, Tetraalkylammoniumcyanohydride, (z. B. Tetrabutylammoniumcyanoborhydrid), Alkalimetallborhydride, z. B. Natriumborhydrid und vorzugsweise Alkalimetallcyanoborhydride, Lithiumcyanoborhydrid und Natriumcyanoborhydrid.

Das Verfahren wird üblicherweise in einem inerten Lösungsmittel durchgeführt. Das Lösungsmittel kann ein organisches oder anorganisches sein, in dem das selektiv geschützte 4,6-Di-O-(aminoglycosyl)-1,3-diaminocyclitol und die anderen Reagentien löslich sind und das unter den Reaktionbedingungen nach Möglichkeit Nebenreaktionen herabselzt oder verhindert. Obwohl wasserfreie aprotische Lösungsmittel mit Vorteil eingesetzt werden können, z. B. Tetrahydrofuran, wenn das Reduktionsmittel Morpholinboran ist, wird gewöhnlich doch ein protisches Lösungsmittel verwendet. Als solches eignet sich z. B. ein niederes Alkanol oder vorzugsweise Wasser oder ein wäßriges niedriges Alkanol, vorzugsweise wäßriges Methanol, Ethanol oder Aceton oder andere Lösungsmittelsysteme, die Wasser enthalten, wie wäßriges Dimethylformamid, wäßriges Hexamethylphosphoramid, wäßriges Tetrahydrofuran oder wäßriger Ethylenglycoldimethylester.

Das Verfahren wird gewöhnlich in einem pH-Bereich von 1 bis 11 und vorzugsweise bei pH 4 bis 8 durchgeführt.

Bei den in dem Verfahren verwendeten Aldehyden handelt es sich um Kohlenhydrate oder Derivate davon. Sie sind in den meisten Fällen durch bekannte Synthesen zugänglich wie sie z. B. in "Methods in Carbohydrate Chemistry", Academic Press-New York and London-Bände I—V beschrieben sind. Sie können entweder in freier Form oder auch als Acetale — z. B. als Dimethylacetale-zur reduktiven Alkylierung eingesetzt werden.

Man arbeitet bei der Verwendung von Acetalen in Gegenwart von Mineralsäuren oder organischen Säuren wie Essigsäure, wodurch das Acetal gespalten wird und der freigesetzte Aldehyd sofort mit der entsprechenden Aminogruppe der Aminotrisaccharidderivate der Formel (II) reagiert.

Ein wichtiger Vorteil der erfindungsgemäßen Verwendung von Kohlenhydraten oder deren Derivaten für die reduktive Einführung von Polyhydroxyalkyl- oder -alkenylresten ist die Tatsache, daß in Form der Zucker eine große Zahl polyfunktioneller und vor allem optisch einheitlicher Aldehydverbindungen für reduktive Alkylierungen zur Verfügung stehen. Dabei ist besonders zu berücksichtigen, daß die reinen Komponenten von Diastereomerengemischen der erfindungsgemäßen Aminoglykosidantibiotika sich in ihren biologischen Eigenschaften meist deutlich voneinander unterscheiden.

Bei den erfindungsgemäß verwendeten Zuckern handelt es sich um Pentosen wie D-Ribose oder

L-Arabinose oder Desoxyderivaten hiervon wie 2-Desoxy-D-ribose oder 5-Desoxy-D-ribose.

Ein weiteres Verfahren zur Herstellung der erfindungsgemäßen Verbindungen, bei welchen der Rest R für einen 2,3,4-Trihydroxypentyl- oder 2,3,4,5-Tetrahydroxypentylrest steht, besteht darin, daß man die Verbindungen der Formel III

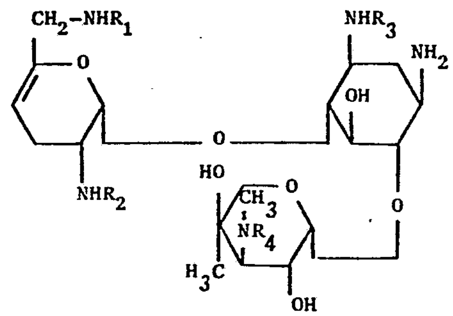

(II)

worin
$R_1$, $R_2$, $R_3$ und $R_4$ die oben angegebene Bedeutung haben,
mil Epoxyverbindungen der Formel IV

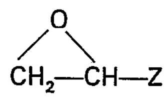

(IV)

wobei
Z für einen 1,2-Dihydroxypropyl- oder 1,2,3-Trihydroxypropylrest steht,
umsetzt und anschließend die vorhandenen Schutzgruppen abspaltet.

Die Erfindung umfaßt ferner Verbindungen der Formel V

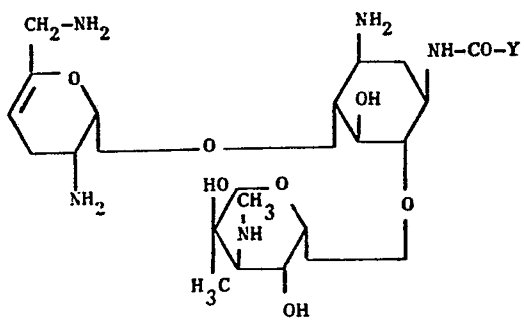

(V)

in welcher
Y die oben für Formel III angegebene Bedeutung aufweist.

Diese Verbindungen sind wertvolle Zwischenprodukte zur Herstellung der Verbindungen der Formel (I), besitzen jedoch ebenfalls ein antibakterielles Breitbandspektrum und eine günstige Verträglichkeit.

Die Verbindungen der Formel V werden erhalten, indem man eine Verbindung der Formel II

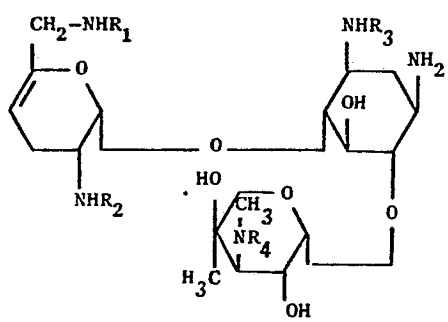

(II)

worin
$R_1$, $R_2$, $R_3$ und $R_4$ die oben angegebene Bedeutung haben, mit einem Säurederivat der Formel VI

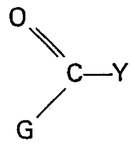

(VI)

wobei
Y die oben angegebene Bedeutung hat und
G entweder eine Hydroxylgruppe bedeutet, wobei die Reaktion in Gegenwart eines Katalysators oder eines wasserentziehenden Mittels durchgeführt wird,
oder eine bei N-Acylierungsreaktionen gebräuchliche Abgangsgruppe, vorzugsweise Halogen oder p-Nitrophenoxy, darstellt,
umsetzt und anschließend die vorhandenen Schutzgruppen abspaltet.

Die Acylierung kann nach den in der Peptidchemie üblichen Verfahren (s. z.B. Houben-Weyl, Methoden der Organischen Chemie, Band XV, Georg Thieme Verlag, Stuttgart, 1974) durchgeführt werden.

Die Acylierung der selectiv geschützten Aminotrisaccharidderivate der Formel (IV) erfolgt vorzugsweise in inerten organischen Lösungsmitteln wie $CHCl_3$, DMF, Pyridin oder Gemischen von solchen Lösungsmitteln mit Alkoholen, vorzugsweise Methanol oder Äthanol.

Als Hilfsbasen können alle in der organischen Chemie üblichen basischen Verbindungen wie z.B. Triäthylamin, Pyridin, Diazabicyclononen oder aber Alkalihydroxide bzw.-carbonate wie Natronlauge oder Natriumcarbonat verwendet werden.

Die Acylierungen werden bei Temperaturen zwischen etwa —30°C und etwa +50°C, vorzugsweise zwischen 0°C und +25°C, durchgeführt.

Die Umsetzungen können sowohl bei Normaldruck, aber auch bei erhöhtem Druck durchgeführt werden. Im allgemeinen arbeitet man bei Normaldruck.

Nach der N-Acylierung werden die im Molekül vorhandenen N- und O-Schutzgruppen in an sich bekannter Weise abgespalten.

Die vorliegende Erfindung umfaßt auch die Verwendung der N-Acylaminotrisaccharide der Formel V als Zwischenprodukte zur Darstellung der entsprechenden Polyhydroxyalkylverbindungen der Formel I, dadurch gekennzeichnet, daß man die Verbindungen der Formel V mit einem Wasserstoff enthaltenden Amid-Reduktionsmittel reagieren läßt und das gewünschte Derivat der Formel I als solches oder als pharmazeutisch annehmbares Säureadditionssalz isoliert.

Das Verfahren wird gewöhnlich in einem inerten organischen Lösungsmittel durchgeführt, worin die Ausgangsverbindungen und das Reduktionsmittel löslich sind und welches Nebenreaktionen nach Möglichkeit unterdrückt. Beispiele für solche Lösungsmittel sind Äther wie Dioxan, Tetrahydrofuran, Diäthylenglykoldimethyläther usw. Bevorzugte Reduktionsmittel sind Aluminiumhydride und Borhydride wie Lithiumaluminiumhydrid, Aluminiumhydrid, Dibroan usw. Im allgemeinen wird bevorzugt Diboran als Reduktionsmittel verwendet. Besitzt jedoch die Ausgangsverbindung eine Doppelbindung, verwendet man bevorzugt Lithiumaluminiumhydrid.

Die erfindungsgemäßen Verbindungen sind antimikrobielle Mittel mit einem breiten Wirkungsspektrum und besonderer Wirksamkeit gegen gram-negative Bakterien. Diese Eigenschaften ermöglichen ihre Verwendung als Arzneimittel, insbesondere bei der Bekämpfung von durch Bakterien hervorgerufenen Erkrankungen bei Mensch und Tier. Sie sind gut zur Prophylaxe und Chemotherapie von lokalen und systemischen Infektionen, insbesondere Infektionen des Urogenitalsystems, in der Human- und Tiermedizin geeignet, die durch gram-negative Bakterien, z.B. E.coli, Proteus, Klebsiella und Pseudomonas verursacht werden. Hemmhöfe im Agar-Lochtest wurden z. B. gegen folgende Bakterienstämme bei einer Konzentration von 100 Mikrogramm/1 ml gefunden.

| | |
|---|---|
| Pseudomonas aerug. | 5737 |
| Pseudomonas aerug. | F 41 |
| Klebsiella pneum. | 2 München |
| Klebsiella pneum. | 1 Düsseldorf |
| E. coli | Münster |
| E. coli | Neumann |

Aus FR—A 2355029 sind bereits Derivate des Kanamycins A bekannt geworden, welche am 1-N-Atom Polyhydroxyalkylreste (beispielsweise den 2,3,4,5-Tetrahydroxypentylrest) tragen. Wie sich zeigte, haben derartige Derivate gegenüber unsubstituiertem Kanamycin A keine nenneswert erhöhte antimikrobielle Wirkung. Im Gegensatz dazu führt die Einführung eines Polyhydroxyalkylsubstituenten

und das 1-N-Atom des Sisomicins zu einer sprunghaften Wirkungssteigerung. Innerhalb der Polyhydroxyalkylreste nehmen in diesem Zusammenhang der Trihydroxy- und Tetrahydroxypentylrest eine herausragende Stellung, beispielsweise im Vergleich mit dem Dihydroxypropylrest, ein.

Im allgemeinen hängt die zu verabreichende Dosis der erfindungsgemäßen Verbindungen vom Alter und Gewicht des Lebewesens, von der Verabreichungsart, von Typ und vom dar Schwere der bakteriellen Infektion ab. Die Dosierung der erfindungsgemäßen Verbindungen ist gewöhnlich der Dosierung der 1-N- unsubstituierten Verbindungen ähnlich. Der Dosierungsspielraum beträgt von 20 mg/-Tag/Mensch bis 2000/mg/Tag/Mensch, vorzugsweise 100 mg — 500 mg/Tag.

Die Verbindungen der Erfindung können oral verabreicht werden. Die Verabreichung kann in Einzelgaben oder auf mehreren Gaben verteilt erfolgen. Sie können auch topisch verabreicht werden in der Form von Salben, Cremen oder Lotionen. Pharmazeutische Trägerstoffe für diese Formulierungen umfassen Wasser, Öle, Fette Polyester und Polyole.

Wie im folgenden gezeigt wird, ist das Verfahren zur Herstellung von Arzneimittel, welche die erfindungsgemäßen Produkte enthalten, dadurch gekennzeichnet, daß man die neuen Verbindungen mit pharmazeutisch geeigneten Träger- und/oder Zusatzstoffen vermischt.

Zur oralen Verabreichung der Verbindungen dieser Erfindung können Tabletten, Kapseln oder Elixiere Anwendung finden, die Verbindungen können aber auch dem Tierfutter zugemischt werden.

Im allgemeinen enthalten topische Präparationen ca. 0,1 bis ca. 3,0 g der Verbindungen der Erfindung pro 100 g Salbe, Creme oder Lotion. Die topische Verabreichung erfolgt ca. 2 bis 5 mal am Tag.

Die antibakteriellen Mittel der Erfindung können in flüssiger Form als Lösungen oder Suspensionen zur Anwendung an Ohren und Augen oder zur parenteralen Verabreichung in Form intramuskulärer Injektionen vorliegen. Injektionslösungen oder -suspensionen werden gewöhnlich so verabreicht, daß ca. 1 bis 15 mg Wirkstoff pro Kilogramm Körpergewicht in 2 bis 4 Dosen pro Tag in den infizierten Organismus gelangen. Die genaue Dosis hängt von der Art der Infektionen, der Empfindlichkeit des infizierenden Keimes und von den individuellen Charakteristika des zu Behandelnden ab.

Formulierungsbeispiel

| Injektionslösung | Per 2,0 ml Viole | per 50 Liter |
|---|---|---|
| 1-N-[(S,S,R)-2,3,4,5-tetrahydroxypentyl]-sisomicin | 84,0 mg ± | 2100.0 gm |
| Methylparaben, U.S.P. | 3,6 mg | 90,0 gm |
| Propylparaben, U.S.P. | 0,4 mg | 10,0 gm |
| Natriumbisulfit, U.S.P. | 6,4 mg | 160 gm |
| Dinatriumäthylendiamintetraacetat-dihydrat | 0,2 mg | 5,0 mg |
| Wasser, U.S.P. q.s. | 2,0 mg | 50,0 Liter |

±5%iger Überschuß

In den folgenden Ausführungsbeispielen wurden zur Ermittlung des Rf-Wertes die folgenden Laufmittelsysteme benutzt:

Laufmittelsystem A = Methylenchlorid:Methanol:20%iger wäßriger Ammoniak (2:4:1)
Laufmittelsystem B = Methylenchlorid:Methanol:konz. Ammoniak (2:2:1)

Die Dünnschichtchromatographie erfolgte auf Kieselgelfertigplatten der Firma Merck, Darmstadt.

Beispiel 1

A) 2'-3,3'',6'-Tetra-N-(o-nitrophenylsulfenyl)-sisomicin

*a) Penta-N-(o-nitrophenylsulfenyl)-sisomicin*

13,84 g (20 mMol) Sisomicinsulfat in 100 ml 1 n NaOH und 450 ml frisch destilliertes Dioxan werden mit 38 g (0,10 Mol) o-Nitrophenylsulfenylchlorid in 200 ml Dioxan und mit 260 ml 1 n-NaOH versetzt, so daß der pH zwischen 12 und 14 liegt. Der Niederschlag wird abfiltriert, in $CH_2Cl_2/H_2O$ gelöst, und die $CH_2Cl_2$-Phase mit $Na_2SO_4$ getrocknet.

Das Filtrat wird mit $CH_2Cl_2$ versetzt, die wäßrige Phase verworfen und die organische Phase über $Na_2SO_4$ getrocknet.

Die vereinigten organischan Phasen werden zur Trockne eingedampft und über 250 g Kieselgel (Säulendurchmesser 8 cm) zuerst mit $CH_2Cl_2$, dann mit $CH_2Cl_2/MeOH = 97,5/2,5$ filtriert. Das Eluat

liefert nach dem Abdampfen des Lösungsmittels 22 g (91%) Penta-N-(o-nitrophenylsulfenyl)-sisomicin als orangefarbenen Schaum.

13-C-NMR ($CDCl_3$):
$\delta$ = 124—148 (arom. H);
102.30 (—1″); 99.00 (C—1′),
97.91 (C—4′); 89.05 (C—6′);
82.33 (C—4); 57.31 (C—1)
56.73 (C—3) ppm.

b) *3″-N-(o-Nitrophenylsulfenyl)-sisomicin*

16.0 g (13,2 mMol) Penta-N-NPS-sisomicin (NPS steht für O-Nitrophenylsulfenyl) in 80 ml absol. Pyridin werden mit 160 ml Thiophenol versetzt, nach 1 Stunde auf 500 ml Diäthyläther gegossen, der Niederschlag in Dichlormethan/Methanol = 8/2 aufgenommen und über Kieselgel filtriert (Säule 5,5 × 12 cm, Laufmittel Dichlormethan/Methanol = 8/2, steigender Zusatz des Laufmittelgemisches Methanol/Dichlormethan/20 proz. Ammoniak = 4/2/1); die rote Zone liefert nach dem Abdampfen des Lösungsmittels 6,6 g (83%) 3″-N-o-Nitrophenylsulfenylsisomicin als tiefroten Schaum.

13-C-NMR($CD_3OD$):
33.59 ($CH_3N$); 52.23 (C—1);
51.16 (C—3); 53 (C—2′)
43.84 (C—6′) ppm.

c) *2′,3,3″,6′-Tetra-N-(o-nitrophenylsulfenyl)-sisomicin und 1,2′,6′-Tetra-N-(o-nitrophenylsulfenyl)-sisomicin*

3,0 g (5,0 mMol) 3″-N-NPS-sisomicin in 5 ml Methanol und 45 ml Dichlormethan werden mit 4,4 g (15,0 mMol) o-Nitrophenylsulfensäure-p-nitrophenylester in 85 ml Dichlormethan versetzt, das Reaktionsgemisch sofort zur Trockne eingedampft, in Dichlormethan aufgenommen und an Kieselgel (Säule 5,5 x 30 cm) mit 200 ml Dichlormethan, dann mit Dichlormethan/Methanol chromatographiert. Es werden 500 Fraktionen aufgefangen, wobei aus den vereinigten Fraktionen 150 bis 250 ds 1,2′,3″,6′-Tetra-NPS-sisomicin und aus den Fraktionen 270 bis 500 das 2′,3,3″,6′-Tetra-NPS-Derivat jeweils als orangefarbener Schaum erhalten wird.

1,2′,3″,6′-Tetra-NPS-sisomicin:
$R_F$ ($CH_2Cl_2/CH_3OH$ = 9/1): 0.62
IR(KBr): 1501, 1360, 1300 (stark); 1587, 1562, 755 (mittel); 1442, 780, 890 (schwach)

2′,3,3″,6′-Tetra-NPS-sisomicin:
$R_F$($CH_2CH/CH_3OH$ = 9/1): 0,42
IR(KBr): 1500, 1358, 1296 (stark); 1586, 1560, 754 (mittel); 1442, 890, 779 (schwach)

B) 1-N-[(R,S,R)-2,3,4,5-Tetrahydroxypentyl]-sisomicin

825 mg 2′,3,3″,6′-Tetra-N-(o-nitrophenylsulfenyl)-sisomicin werden in 16 ml Aceton und 3,75 ml Wasser gelöst. Diese Lösung versetzt man mit 800 ml D-Arabinose und erhitzt 30 Minuten auf 75°C. Nun fügt man 250 mg Natriumcyanoborhydrid und erhitzt für weitere 2 Stunden. Man arbeitet wie in Beispiel 7 beschrieben auf und reinigt das gewünschte Zwischenprodukt durch Säulenchromatographie an Kieselgel mit dem Elutionsmittel Methylenchlorid/Methanol (9:1). Man erhält 1 - N - [(R,S,R) - 2,3,4,5 - Tetrahydroxypentyl] - 2,3,3″,6′ - Tetra - N - (o - nitrophenylsulfenyl) - sisomicin als orangefarbenen Farbstoff. Zur Abspaltung der Schutzstoffgruppen löst man das Produkt in Methylenchlorid, versetzt diese Lösung mit einer gesättigten lösung von Schwefelwasserstoff in Methanol und säuert mit Salzsäure an, Man extrahiert den Wirkstoff mit Wasser, wäscht die wässrige Phase 2 mal mit Methylenchlorid, entionisiert sie mit basischem Ionenaustauscher und isoliert die gewünschte Verbindung durch Gefriertrocknen. $[\alpha]_D^{22} = +99°$ (C = 1.0 $H_2O$). Auf die gleiche Weise und unter Verwendung von 2′,3,3″,6′ - Tetra - N - (o - nitrophenylsulfenyl) - sisomicin und den jeweils angegebenen Hydroxyaldehyden erhält man die folgenden Verbindungen:

Aus D-Ribose 1 - N - [(R,R,S) - 2,3,4,5 - Tetrahydroxypentyl] - 2′,3,3″,6′ - Tetra - N - (o - nitrophenylsulfenyl) - sisomicin mit $[\alpha]_D^{22} = +64°$ (C = 1,0 DMSO) und daraus 1 - N - [(R,R,S) - 2,3,4,5 - tetrahydroxypentyl] - sisomicin, Rf-Wert 0,42 (Laufmittelsystem B).

Aus L-Arabinose 1 - N - [(S,R,S) - 2,3,4,5 - Tetrahydroxypentyl] - 2′,3,3″,6′ - tetra - N - (o - nitrophenylsulfenyl) - sisomicin $[\alpha]_D^{20} = +88°$ (C = 1,0 DMSO) und daraus 1 - N - [(S,R,S) - 2,3,4,5 - Tetrahydroxypentyl] - sisomicin $[\alpha]_D^{20} = +95°$ (C = 1,0 $H_2O$).

Aus 2-Desoxy-D-ribose 1 - N - [(S,R) - 3,4,5 - Trihydroxypentyl] - 2′,3,3″,6′ - tetra - N - (o - nitrophenylsulfenyl) - sisomicin $[\alpha]_D^{22} = +74°$ (C = 1,0 DMSO) und daraus 1 - N - [(S,R) - 3,4,5 - Trihydroxypentyl] - sisomicin $[\alpha]_D^{22} = +21°$ (C = 1,0 $CH_3OH$).

Aus D-Xylose 1 - N - [(S,R,R) - 2,3,4,5 - Tetrahydroxypentyl] - 2′,3,3″,6′ - tetra - N - (o -

nitrophenylsulfenyl) - sisomicin $[\alpha]_D^{22} = +49°$ (C = 1,0 DMSO) und daraus 1 - N - [(S,R,R) - 2,3,4,5 - Tetrahydroxypentyl] - sisomicin, Rf-Wert = 0,3 (Laufmittelsystem B).

**Patentansprüche**

1. 4,6-Di-O-(Aminoglycosyl)-1,3-Diaminocyclitol-Derivate der allgemeinen Formel I

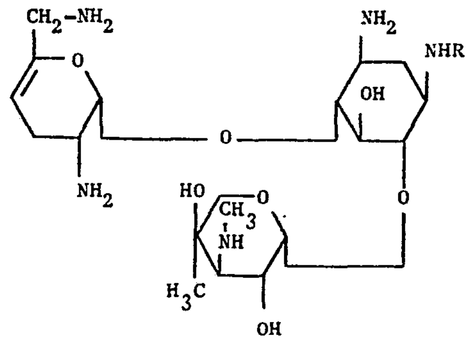

(I)

worin
R einen 2,3,4-Trihydroxypentyl-, 3,4,5-Trihydroxypentyl- oder 2,3,4,5-Tetrahydroxypentylrest bedeutet.

2. 1-N-(2,3,4,5-Tetrahydroxypentyl)-sisomicin.

3. 1-N-[(R,R,S)-2,3,4,5-Tetrahydroxypentyl]-sisomicin.

4. Verfahren zur Herstellung von Verbindungen gemäß Anspruch 1 bis 3, dadurch gekennzeichnet, daß man eine Verbindung der Formel II

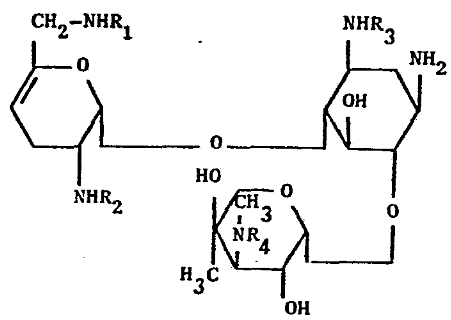

(II)

worin
$R_1$, $R_2$, $R_3$ and $R_4$ eine Gruppe —SR′ oder —CO—A′ bezeichnen, wobei
R′ für eine gegebenenfalls substituierte Phenyl-, Di- oder Triphenylmethylgruppe und
A′ für einen Rest

$-(CH_2)_{n_1}-B$ oder 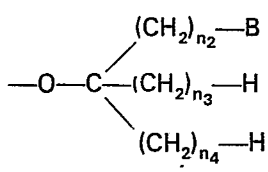

stehen,
B Wasserstoff oder eine gegebenenfalls substituierte Phenylgruppe bedeutet, und
$n_1$, $n_2$, $n_3$ und $n_4$ unabhängig voneinander Zahlen von 0 bis 5 darstellen,
mit einem Aldehyd der Formel III

$$HOC-Y, \qquad (III)$$

wobei
Y für einen 1,2,3-Trihydroxybutyl-, 2,3,4-Trihydroxybutyl- oder 1,2,3,4-Tetrahydroxybutylrest steht, in Gegenwart eines Wasserstoffdonor-Reduktionsmittels umsetzt und anschließend vorhandenen Schutzgruppen abspaltet.

5. Verfahren zur Herstellung von Verbindungen gemäß Anspruch 1, bei denen der Rest R für einen 2,3,4-Trihydroxypentyl- oder 2,3,4,5-Tetrahydroxypentylrest steht, dadurch gekennzeichnet, daß man Verbindungen der Formel II

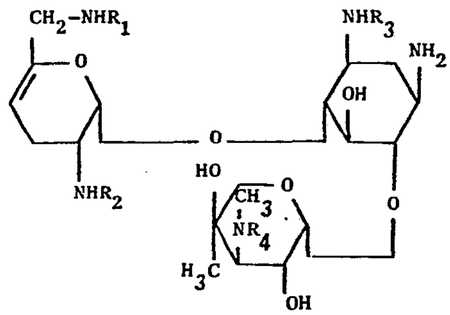

(II)

worin
$R_1$, $R_2$, $R_3$ und $R_4$ die in Anspruch 4 angegebene Bedeutung haben, mit Epoxyverbindungen der Formel IV

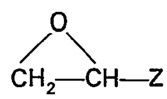

(IV)

wobei
Z für einen 1,2-Dihydroxypropyl- oder 1,2,3-Trihydroxypropylrest steht, umsetzt und anschließend die vorhandenen Schutzgruppen abspaltet.

6. Verbindungen der allgemeinen Formel V

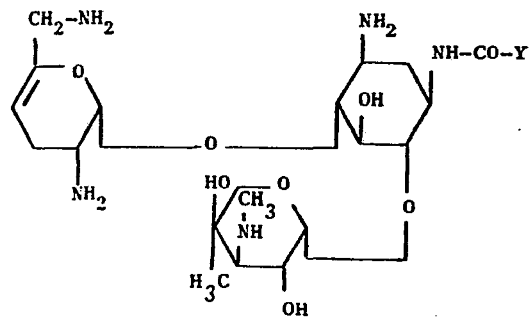

(V)

in welcher
Y die in Anspruch 4 angegebene Bedeutung aufweist.

7. Verfahren zur Herstellung von Verbindungen gemäß Anspruch 6, dadurch gekennzeichnet, daß man eine Verbindung der Formel II

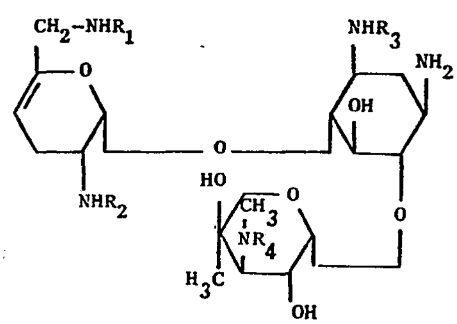

(II)

worin
$R_1$, $R_2$, $R_3$ und $R_4$ die in Anspruch 4 angegebene Bedeutung haben, mit einem Säurederivat der Formel VI

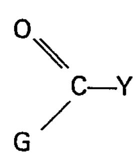

(VI)

wobei
Y die in Anspruch 4 angegebene Bedeutung hat und

G entweder eine Hydroxylgruppe bedeutet, wobei die Reaktion in Gegenwart eines Katalysators oder eines wasserentziehenden Mittels durchgeführt wird,
oder eine bei N-Acylierungsreaktionen gebräuchliche Abgangsgruppe, vorzugsweise Halogen oder p-Nitrophenoxy, darstellt,
umsetzt und anschließend die vorhandenen Schutzgruppen abspaltet.

8. Verfahren zur Herstellung von Verbindungen gemäß Anspruch 1 bis 3, dadurch gekennzeichnet, daß man Verbindungen gemäß Anspruch 6 mit einem wasserstoffenthaltenden Reduktionsmittel reduziert.

9. Arzneimittel, gekennzeichnet durch einen Gehalt an mindestens einer Verbinding der Ansprüche 1 bis 3 oder 6.

10. Verfahren zur Herstellung antimikrobieller Mittel, dadurch gekennzeichnet, daß man wenigstens eine Verbindung der Ansprüche 1 bis 3 oder 6 mit inerten, nichttoxischen, pharmazeutisch geeigneten Trägerstoffen vermischt.

## Claims

1. 4,6-Di-O-(aminoglycosyl)-1,3-diaminocyclitol derivatives of the general formula I

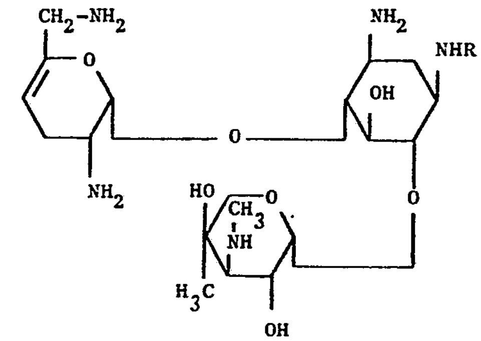

(I)

wherein
R denotes a 2,3,4-trihydroxypentyl, 3,4,5-trihydroxypentyl or 2,3,4,5-tetrahydroxypentyl radical.

2. 1-N-(2,3,4,5-tetrahydroxypentyl)-sisomicin.

3. 1-N-[(R,R,S)-2,3,4,5-tetrahydroxypentyl]-sisomicin.

4. Process for the production of compounds according to Claim 1 to 3, characterised in that a compound of the formula II

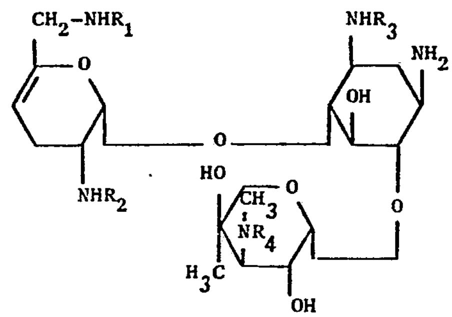

(II)

wherein
$R_1$, $R_2$, $R_3$ and $R_4$ denote a group —SR′ or —CO—A′, wherein
R′ represents an optionally substituted phenyl, diphenylmethyl or triphenylmethyl group and A′ represents a radical

$-(CH_2)_{n_1}-B$ oder 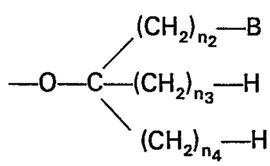

B denotes hydrogen or an optionally substituted phenyl group, and
$n_1$, $n_2$, $n_3$ and $n_4$ independently of one another represent numbers from 0 to 5,
is reacted with an aldehyde of the formula III

$$HOC-Y, \qquad (III)$$

wherein

Y represents a 1,2,3-trihydroxybutyl, 2,3,4-trihydroxybutyl or 1,2,3,4-tetrahydroxybutyl radical, in the presence of a hydrogen donor reducing agent and the protective groups present are then split off.

5. Process for the production of compounds according to Claim 1 in which the radical R represents a 2,3,4-trihydroxypentyl or 2,3,4,5-tetrahydroxypentyl radical, characterised in that compounds of the formula II

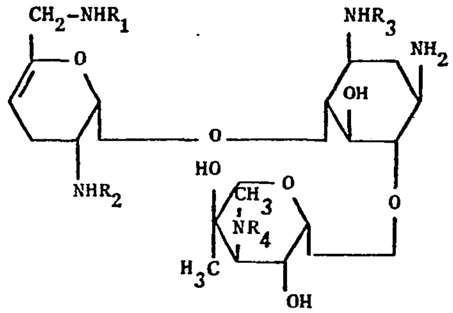

(II)

wherein

$R_1$, $R_2$, $R_3$ and $R_4$ have the meaning indicated in Claim 4, are reacted with epoxy compounds of the formula IV

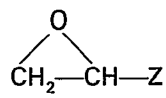

(IV)

wherein

Z represents a 1,2-dihydroxypropyl or 1,2,3-trihydroxypropyl radical, and the protective groups present are then split off.

6. Compounds of the general formula V

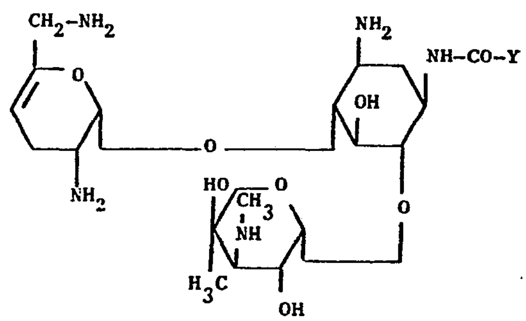

(V)

in which

Y has the meaning indicated in Claim 4.

7. Process for the production of compounds according to Claim 6, characterised in that a compound of the formula II

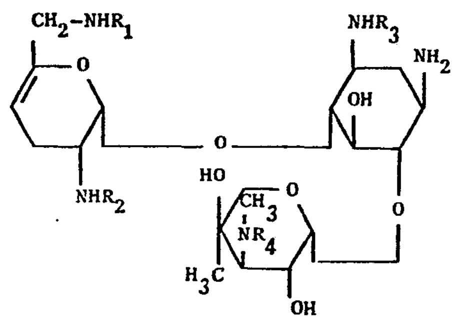

(II)

wherein

$R_1$, $R_2$, $R_3$ and $R_4$ have the meaning indicated in Claim 4, is reacted with an acid derivative of the formula VI

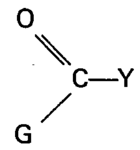

(VI)

wherein

Y has the meaning indicated in Claim 4 and

G either denotes a hydroxyl group, in which case the reaction is carried out in the presence of a catalyst or a dehydrating agent, or represents an N-acylation reaction leaving group, preferably halogen or p-nitrophenoxy,

and the protective groups present are then split off.

8. Process for the production of compounds according to Claim 1 to 3, characterised in that compounds according to Claim 6 are reduced with a hydrogen-containing reducing agent.

9. Medicaments, characterised in that they contain at least one compound according to Claims 1 to 3 or 6.

10. Process for the preparation of antimicrobial compositions, characterised in that at least one compound of Claims 1 to 3 or 6 is mixed with inert, non-toxic, pharmaceutically suitable excipients.

**Revendications**

1. Dérivés de 4,6-di-O-(aminoglycosyl)-1,3-diaminocyclitols de formule générale I:

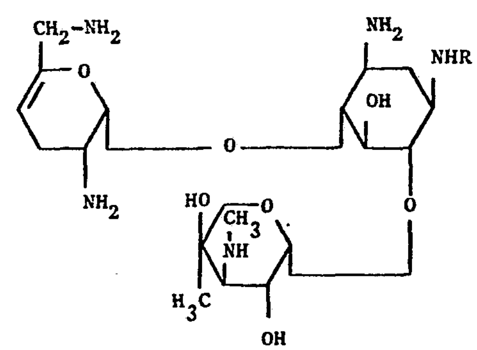

(I)

dans laquelle

R représente un groupe 2,3,4-trihydroxypentyle, 3,4,5-trihydroxypentyle ou 2,3,4,5-tétrahydroxypentyle.

2. La 1-N-(2,3,4,5-tétrahydroxypentyl)-sisomycine.

3. La 1-N-[(R,R,S)-2,3,4,5-tétrahydroxypentyl]-sisomycine.

4. Procédé de préparation de composés suivant les revendications 1 à 3, caractérisé en ce qu'on fait réagir un composé de formule II:

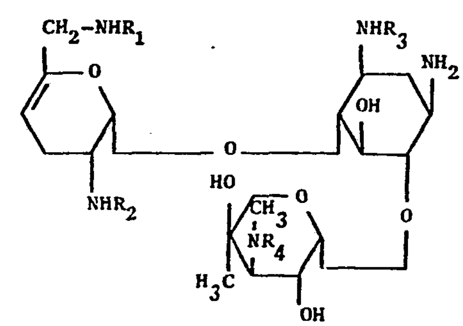

(II)

dans laquelle

$R_1$, $R_2$, $R_3$ et $R_4$ représentent chacun un groupe —SR′ ou —CO—A′ où

R′ représente un groupe triphénylméthyle, diphénylméthyle ou phényl éventuellement substitué et

A′ représente un radical

$—(CH_2)_{n_1}—B$ ou 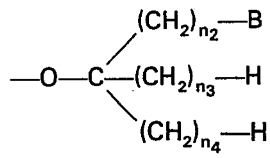

B représente un atome d'hydrogène ou un groupe phényle éventuellement substitué, et $n_1$, $n_2$, $n_3$ et $n_4$ représentent indépendamment l'un de l'autre des nombres de 0 à 5, avec un aldéhyde de formule III:

$$HOC—Y \quad (III)$$

où

Y représente un groupe 1,2,3-trihydroxybutyle, 2,3,4-trihydroxybutyle ou 1,2,3,4-tétrahydroxybutyle, en présence d'un agent réducteur donneur d'hydrogène, puis on sépare les groupes protecteurs présents.

5. Procédé de préparation de composés suivant la revendication 1 dans lesquels le radical R represénte un groupe 2,3,4-trihydroxypentyle ou 2,3,4,5-tétrahydroxypentyle, caractérisé en ce qu'on fait réagir des composés de formule II:

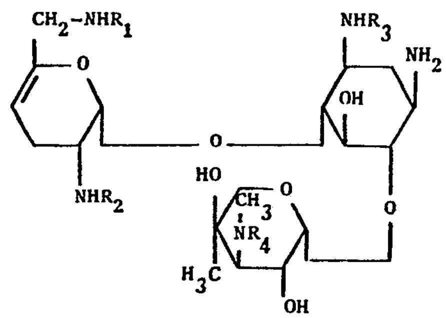

(II)

dans laquelle

$R_1$, $R_2$, $R_3$ et $R_4$ ont les significations indiquées dans la revendication 4, avec des composés époxy de formule IV:

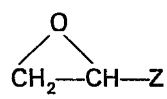

(IV)

où

Z représente un groupe 1,2-dihydroxypropyle ou 1,2,3-trihydroxypropyle, puis on sépare les groupes protecteurs présents.

6. Composés de formule générale V:

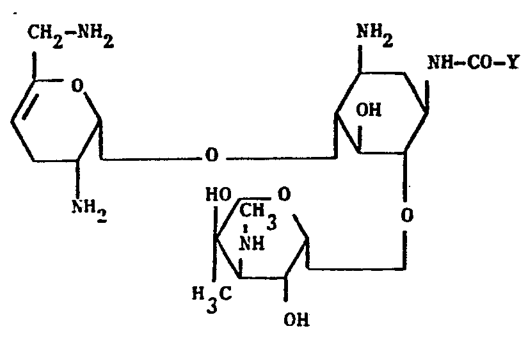

(V)

dans laquelle

Y a la signification indiquée dans la revendication 4.

7. Procédé de préparation de composés suivant la revendication 6, caractérisé en ce qu'on fait réagir un composé de formule II:

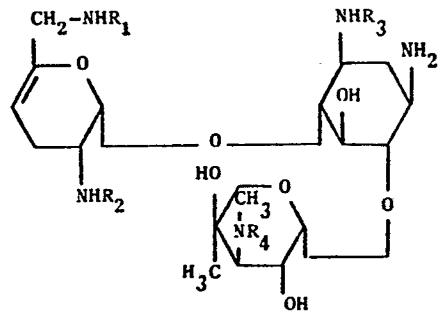

(II)

dans laquelle

$R_1$, $R_2$, $R_3$ et $R_4$ ont les significations indiquées dans la revendication 4,

avec un dérivé d'acide de formule VI:

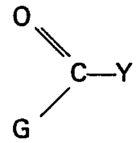

(VI)

où

Y a la signification indiquée dans la revendication 4 et

G représente soit un groupe soit un groupe hydroxy, la réaction étant alors effectuée en présence d'un catalyseur ou d'un agent déshydratant, soit un groupe de départ habituellement utilisé dans des réactions de N-acylation, de préférence, un atome d'halogène ou un groupe p-nitrophénoxy, puis on sépare les groupes protecteurs présents.

8. Procédé de préparation de composés suivant les revendications 1 à 3, caractérisé en ce qu'on réduit des composés suivant la revendication 6 avec un agent réducteur contenant de l'hydrogène.

9. Médicaments, caractérisés en ce qu'ils contiennent au moins un composé suivant les revendications 1 à 3 ou 6.

10. Procédé de préparation d'agents antimicrobiens, caractérisé en ce qu'on mélange au moins un composé suivant les revendications 1 à 3 ou 6 avec des substances supports inertes, non toxiques et pharmaceutiquement appropriées.